Europäisches Patentamt

European Patent Office

Office européen des brevets

(19)

(11) Publication number : **0 419 282 B1**

(12) **EUROPEAN PATENT SPECIFICATION**

(45) Date of publication of patent specification :
21.07.93 Bulletin 93/29

(51) Int. Cl.⁵ : **A61L 2/26, C12Q 1/22**

(21) Application number : **90310367.9**

(22) Date of filing : **21.09.90**

(54) **Disposable test packs for steam or gas sterilizers.**

(30) Priority : **22.09.89 US 410973**

(43) Date of publication of application :
**27.03.91 Bulletin 91/13**

(45) Publication of the grant of the patent :
**21.07.93 Bulletin 93/29**

(84) Designated Contracting States :
**DE FR GB IT**

(56) References cited :
**No relevant documents have been disclosed.**

(73) Proprietor : **MINNESOTA MINING AND
MANUFACTURING COMPANY
3M Center, P.O. Box 33427
St. Paul, Minnesota 55133-3427 (US)**

(72) Inventor : **Hart, Marvin L., Minnesota Mining &
Manufact. Co.
2501 Hudson Road, P.O. Box 33427
St. Paul, Minnesota 55133-3427 (US)**
Inventor : **Kirckof, Steven S. Minnesota Mining
& Manufact.Co.
2501 Hudson Road, P.O. Box 33427
St. Paul, Minnesota 55133-3427 (US)**

(74) Representative : **Baillie, Iain Cameron et al
c/o Ladas & Parry, Altheimer Eck 2
W-8000 München 2 (DE)**

## Description

BACKGROUND OF THE INVENTION

This invention relates to disposable test packs for determining the efficacy of a sterilization cycle in sterilizers, such as, prevacuum or gravity steam sterilizers, or ethylene oxide type sterilizers.

The steam or gas sterilization process used to sterilize medical and hospital equipment cannot be effective unless the steam or gas sterilants have been in contact with all surfaces of the materials being sterilized for the proper amount of time and at the proper temperature. In a prevacuum steam sterilizer, air is removed from the sterilization chamber prior to the introduction of steam. Any air which is not removed from the sterilizer during the prevacuum phase of the cycle or which leaks into the sterilizer after a vacuum is drawn due to faulty gaskets, valves or seals, can form air pockets. These air pockets will create a barrier thereby preventing the materials being sterilized from coming into intimate contact with the steam. This is particularly true when porous materials such as hospital linens or fabrics are being sterilized since the air pockets prohibit the steam from reaching the interior layers of such materials. As a result, sterilization may not occur. In gravity steam sterilizers, incoming steam must effectively displace air in the materials being sterilized in order to provide an adequate sterilization cycle. If the air is not sufficiently displaced by the steam, the air will prevent the materials being sterilized from coming into intimate contact with the steam thereby resulting in inadequate sterilization conditions. In ethylene oxide sterilization, ethylene oxide gas (optionally in combination with other inert, diluent gases such as carbon dioxide and halocarbon gases) is introduced under negative pressure and allowed to mix with water vapor in the sterilization chamber. If the proper conditions (e.g. concentration of ethylene oxide, relative humidity, time and temperature) are not met in the sterilization chamber, the ethylene oxide will not penetrate the materials being sterilized and will not perform as an effective sterilant. Therefore, there is a need for a device for determining the efficacy of sterilization cycles in prevacuum or gravity steam sterilizers, or ethylene oxide sterilizers, which operates by detecting sufficient sterilant penetration with a high degree of reliability and sensitivity.

One commonly used procedure for determining the efficacy of prevacuum steam sterilizers is known as the Bowie-Dick test. A publication by the Association for the Advancement of Medical Instrumentation (AAMI) in AAMI SSSA-1988 entitled "Good Hospital Practice: Steam Sterilization and Sterility Assurance" (1988) prescribed the standard use of the Bowie-Dick type test pack for detecting residual air in prevacuum type sterilizers. The typical Bowie-Dick type test pack essentially consists of a stack of freshly laundered towels folded to a specific size. A chemical indicator sheet is then placed in the center of the pack. If the air removal within the sterilizer is insufficient, an air pocket will form in the center of the pack thereby preventing steam from contacting the steam sensitive chemical indicator sheet. The presence of the air pocket will be recorded by the failure of the indicator sheet to undergo a complete or uniform color change, indicative of adequate air removal.

The Bowie-Dick type test, although generally recognized as an adequate procedure for determining the efficacy of prevacuum steam sterilizers, presents many disadvantages. Since the test pack is not pre-assembled, it must be constructed every time the procedure is used to monitor sterilizer performance. This may interject some inconsistency in the test procedure since varying factors, such as, laundering, pre-humidification, towel thickness and wear, and the number of towels used, alter the color change in the indicator. Also, the preparation and use of this test pack is more time-consuming than a pre-assembled test pack indicator.

There are pre-assembled air indicating test packs known in the prior art, such as the one disclosed in Dyke et al., U.S. Patent No. 4,594,223. The Dyke device includes an elongated tube with a hole at the top into which the steam enters in a downward direction, a heat sink disposed within the elongated tube for condensing the steam and releasing the noncondensable gases, a housing defining a chamber releasably connected to the lower end of the tube, an indicator strip suspended within the chamber, and a semipermeable membrane preventing the condensate from the heat sink from reaching the chamber. The Dyke patent requires that the indicator be housed in a separate chamber so that the condensate will not affect the indicator strip. The Dyke patent also requires the use of a liquid impermeable membrane between the housing and the chamber to further prevent the condensate from coming into contact with the indicator strip. Furthermore, the steam flow as described in the Dyke patent is limited to the downward passage of steam through the heat sink so that the steam does not work against the force of gravity.

A device to monitor the presence of noncondensable gases in steam-gas sterilizers is disclosed in an article in the Journal of Clinical Pathology, Vol. 26, p. 716 (1973) entitled "Testing A Steam-Formaldehyde Sterilizer For Gas Penetration Efficiency" by Stuart J. Line and J.K. Pickerill. The device includes a gas tight brass capsule containing a biological and/or chemical indicator. The capsule is fitted at its upper end with an "0" ring seal which is secured by a knurled ring. A stainless steel helical tubing is used as a challenge to the penetration of the sterilant. The materials used in this device are very expensive. Furthermore, the helix must be cleaned after use by drawing air through it for several minutes. Thus, the device is not as easy to use as pre-assembled, disposable type test packs.

This invention provides disposable test packs for determining the efficacy of a sterilization cycle in prevacuum or gravity steam sterilizers and ethylene oxide sterilizers. The disposable test packs of this invention are pre-assembled, easy to use, small and convenient to handle. These disposable test packs incorporate either chemical or biological indicators which are not affected by condensed steam and, therefor, do not need to be kept in a separate container apart from the packing material, or require a condensate impermeable membrane separating them.

## SUMMARY OF THE INVENTION

The invention provides disposable test packs which determine the efficacy of a sterilization cycle by providing a challenge to the penetration of the sterilant in prevacuum or gravity steam sterilizers, or ethylene oxide sterilizers, and which are relatively inexpensive to manufacture and which are constructed in such a manner that makes them easier to use in comparison to similar types of prior art test packs.

One embodiment of the present invention provides a disposable test pack which includes a vertically extending container made of gas and liquid-impermeable material. The container has a first end wall, a second end wall opposite said first end wall, and sidewalls separating the first end wall from the second end wall. The container has a volumetric capacity in the range of about 2.54 cm$^3$ to 508 cm$^3$. The first end wall has at least one hole having an area of between 0.0254 cm$^2$ and 7.62 cm$^2$ through it for the ingress of sterilant. The second end wall is comprised of removeable means for opening the container. The container is at least partially filled with a porous, fibrous packing material, such as compressed, divellicated polypropylene blown microfiber microweb which challenges the penetration of the sterilant by defining a restricted pathway which impedes the flow of sterilant through the container during the sterilization cycle. The packing material has a "packing factor" characteristic (defined hereinafter) in the range of 2.4 x 10$^{-8}$ cm$^2$ to 1.3 x 10$^{-5}$ cm$^2$. An indicator for detecting sterilant penetration and the extent thereof is provided between the removable lid and the packing material. The removable means provides ready access to the indicator after the test pack has been subjected to a sterilization cycle in a sterilization chamber.

Another embodiment of the present invention provides for a disposable test pack which includes a vertically extending container with a bottom wall portion having at least one hole therethrough for the ingress of the sterilant. The container is at least partially filled with a porous, fibrous mass packing material, such as compressed divellicated polypropylene blown microfiber microweb, which acts as a challenge to the penetration of the sterilant by defining a restricted pathway which acts to impede the flow of the sterilant through the disposable test pack. The top wall of the container is comprised of a lid which seals the upper portion of the container. An indicator for detecting sterilant penetration and the extent thereof is provided adjacent the lid, on the top of the packing material opposite the hole in the bottom wall. The lid comprising the top wall of the container can be easily removed to provide access to the indicator following a sterilization cycle.

An unsuccessful sterilization cycle may occur as a result of various reasons. Since steam is used as the sterilant in a prevacuum sterilizer, it must penetrate the restricted pathway of the packing material. The packing material draws latent heat from the incoming steam which is condensed and adsorbed on the fibrous mass of the packing material. As the steam is condensed and the temperature of the packing material increases, incoming steam further penetrates the packing material. If any noncondensable gas is present in the packing material of the test pack, it will act to inhibit the steam from penetrating through the packing material. Steam will also fail to penetrate the packing material if the sterilization cycle is not operated for the proper length of time at the proper temperature. Furthermore, if a gravity sterilizer is used, the steam must penetrate the packing material and adequately displace the resident and/or entrapped air. If the gravity sterilization cycle is not properly operated for the necessary time at the necessary temperature, total displacement of the air by the steam, vital to an efficacious sterilization cycle, will not occur. Additionally, if ethylene oxide is used as a sterilant, proper conditions must be present in order for the ethylene oxide to properly penetrate the packing material and effectively perform as a sterilant. Any of these fault conditions will be detected by the disposable test pack of the present invention.

In another embodiment of the present invention, a disposable test pack is provided which includes a vertically extending container with a removable lid comprising a first wall and a second wall opposite the first wall which has a hole for the ingress of the steam or gas sterilant Two small openings can be provided on the second wall near the edges as an added safety feature to vent the sterilant which may otherwise burst from the container when the lid is removed. The disposable test pack container is filled with a packing material, such as the compressed divellicated polypropylene blown microfiber microweb used in the other embodiments. A steam chemical indicator sheet, or an ethylene oxide chemical indicator sheet if ethylene oxide is used as the sterilizing gas, is positioned in the container between the packing material and the lid. As the sterilant enters the hole in the first wall of the container, it encounters the packing material which provides a challenge by defining a restricted pathway which acts to impede the penetration of the sterilant. If the sterilant successfully penetrates the packing material of the test pack, the chemical indicator sheet will undergo a complete color change. However, if the sterilant did not sufficiently penetrate the packing material, due to such factors as the presence of noncondensable gases, inadequate displacement of the air by the sterilant in the case of gravity sterilizers, or the failure to operate the sterilizer for the necessary time at the necessary temperature, the chemical indicator will not undergo a completely uniform color change, thereby indicating an unsuccessful sterilization cycle.

BRIEF DESCRIPTION OF THE DRAWINGS

Fig. 1 is an exploded perspective view of a disposable test pack containing a biological indicator.

Fig. 2 is a cross-sectional view along line 2 in Fig. 1 of the disposable test pack, showing the packing material and the biological indicator disposed on top.

Fig. 3 is a top view of the disposable test pack of Fig. 1, shown with the lid removed to reveal the biological indicator disposed within the recess of the plug.

Fig. 4 is a bottom view of the disposal test pack of Fig. 1, showing the holes in the bottom wall.

Fig. 5 is an exploded perspective view of an embodiment of the invention containing the chemical indicator sheet, with the packing material exposed by a cross-sectional view of the container.

Fig. 6 is top view of the test pack in Fig. 5, showing the hole on the top wall of the container providing access to the packing material, and the two steam vent openings.

Fig. 7 is a bottom view of the pack in Fig. 5 showing the removable lid comprising the bottom wall.

DESCRIPTION OF THE PREFERRED EMBODIMENTS

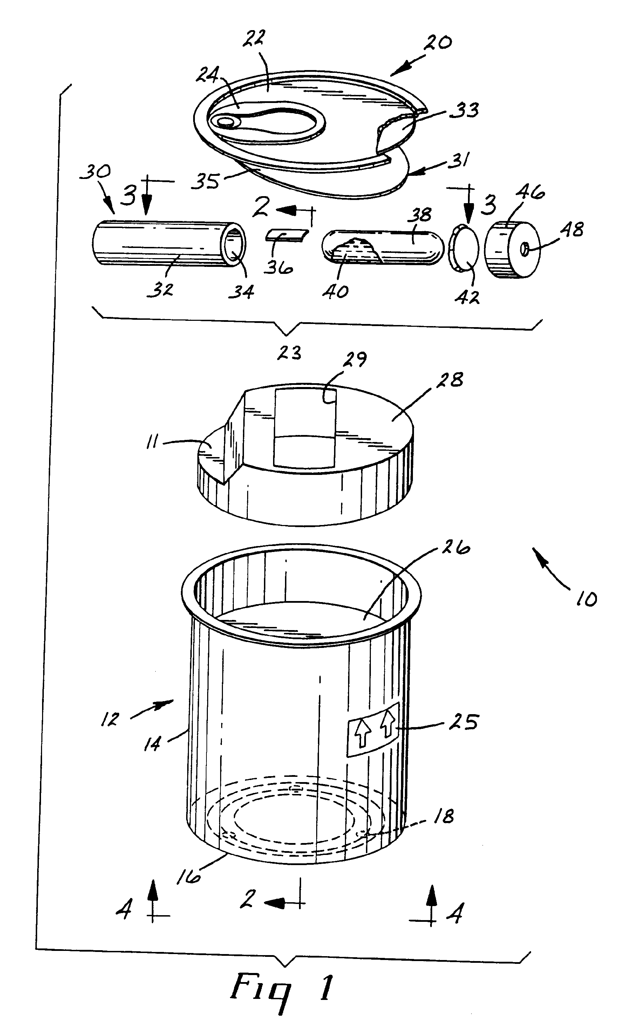

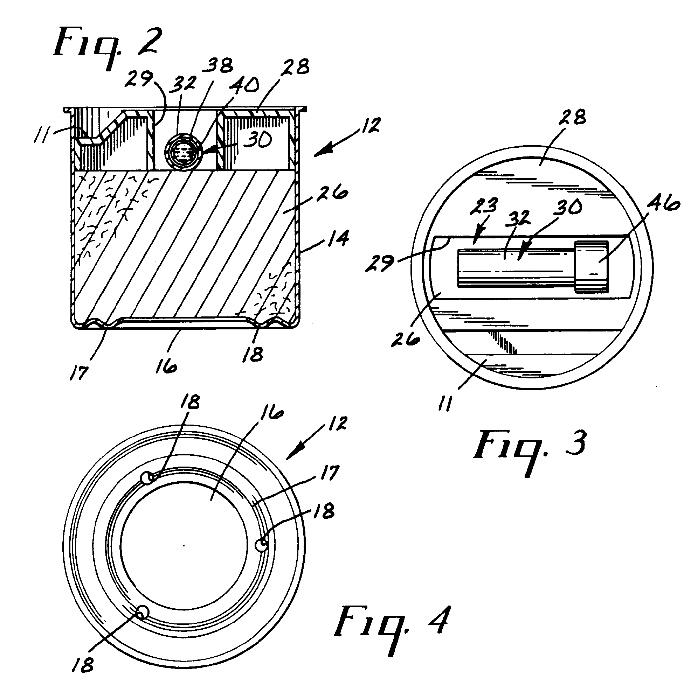

A preferred embodiment of the disposable test pack of the present invention is shown in Figs. 1-4, as generally indicated at 10. The disposable test pack includes a vertically extending container 12 having a tubular side wall 14 integral with a bottom wall 16. Bottom wall 16 has holes 18 for the ingress of sterilant. The container is preferably 3.81 cm (1 1/2 in) in height and 6.35 cm (2 1/2 in) in diameter, designated by size 108 x 208 and commercially available from Central States Can Co., Massillon, Ohio. The container of the present invention preferably has a volumetric capacity in the range of 16.4 cm$^3$ (1 in$^3$) to 3,540 cm$^3$ (216 in$^3$), more preferably in the range of 65.6 cm (4 in$^3$) to 1,312cm$^3$ (80 in$^3$), and most preferably in the range of 114.8 cm$^3$ (7 in$^3$) to 229.6 cm$^3$ (14 in$^3$). The container is preferably a seamless aluminum can. This type of container is relatively inexpensive, small, and easy to handle and use. However, other containers made of rigid gas and liquid-impermeable materials, such as metal, glass, film or metal laminated chip board, polypropylene, polyamides, polymethylpentenes, polyesters and polymethylmethacrylate are also useful. The bottom wall 16 of the container is provided with an outwardly protruding circular ridge 17. The holes 18 are preferably placed in ridge 17. This prevents holes 18 from being blocked by the packing material within the can. Preferably there are three holes 18 which are equidistant from each other and have a diameter of 0.32 cm (1/8 in). Alternatively, the device may include fewer or larger numbers of holes 18, provided that the cumulative areas of the holes are between 0.079 cm$^2$ (0.012 in$^2$) and 20.25 cm$^2$ (3.14 in$^2$), preferably between 0.237 cm$^2$ (0.036 in$^2$) and 5.06 cm$^2$ (0.785 in$^2$). The top wall 20 of the container is comprised of a removable lid 22 sealing the upper portion of the container. The lid has a tab ring 24 for easily removing the lid and providing quick access to the inside of the disposable test pack container. The test pack is provided with a label having arrows 25 on its exterior to show the vertical orientation in which it is to be used.

The test pack container 12 is filled with a porous, fibrous mass packing material 26 for challenging the penetration of the sterilant by defining a restricted pathway which acts to impede the flow of the sterilant through the container. While the amount of packing material varies depending on the packing factor (discussed herein below) and the particular sterilization indicator used, the container is normally filled with packing material to at least one-half its volume. Suitable materials which provide an adequate challenge can be used as packing materials in the disposable test pack of the present invention. Some packing materials found to be suitable include: copy paper commercially available from the assignee of this application, Minnesota Mining and Manufacturing Co. ("3M") of St. Paul, Minnesota, as "Type 696 White Bond Copy Paper"; blotter paper commercially available as "Sorg Bluebird Blotter Paper" from Sorg Paper Products, Middletown, Ohio; polyester/cellulose fiber web commercially available as "Assure™ Nonwoven Web" from Dexter, Windsor Locks, Connecticut; 50/50 blend of rayon/polyester fiber commercially available from BASF Corp., Enka, North Carolina as "Merge 8645 Rayon Staple Fiber"; polyester fiber commercially available from Unitika Ltd., Osaka, Japan as "Melty$^R$ Fiber Type 4080 Polyester"; and a 50/50 blend of cotton and rayon fiber, the rayon fiber commercially available from the BASF Corp. as "Merge 8645 Rayon Staple Fiber". However, the packing materials of the present invention are preferably formed from polyolefin fibers such as polyethylene, polypropylene, polybutylene, or copolymers of ethylene, propylene and/or butylene or blends thereof. The fibers are preferably less than about 50 microns, more preferably less than about 25 microns, and most preferably less than about 15 microns in diameter. The fibers are preferably prepared by melt blowing, flash spinning, or fibrillation. Particularly preferred are blown microfibers in web form which have been milled or divellicated. The packing materials of the invention are preferably microfiber microwebs prepared from source microfiber webs such as, for example, those disclosed in Wente, Van A., "Superfine Thermoplastic Fibers," Industrial and Engineering Chemistry, vol. 48, pp. 1342-1346, (1956) and in Wente, Van A. et al., "Manufacture of Superfine Organic Fibers" Report No. 4364 of the Naval Research Laboratories, published May 25, 1954, or from microfiber webs containing particulate matter such as those disclosed, for example, in U.S. Patent No. 3,971,373 (Braun), U.S. Patent No. 4,100,324 (Anderson et al.), and U.S. Patent No. 4,429,001 (Kolpin et al.).

The preferred packing materials of the invention are formed by compressing the polyolefin fibers to a solidity of at least 20%, preferably at least 30%. The solidity of the packing materials is calculated according to the formula:

$$\% \text{ solidity} = \frac{\text{density of packing materials}}{\text{density of polyolefin in packing materials}} \times 100$$

When the polyolefin fibers are provided as microfiber microwebs, the solidity of the packing material is most preferably 60 to 70%. This provides packing material which can be drilled or milled to the desired shape.

A particularly preferred packing material is comprised of compressed polypropylene blown microfiber. This type of packing material provides an excellent challenge to the penetration of the sterilant in the test packs and thus provides improved test pack sensitivity for determining the efficacy of the sterilization cycle. The preferred compressed polypropylene blown microfiber packing material is made by first preparing a melt blown microfiber web as described in Wente, Van A., "Superfine Thermoplastic Fibers," Industrial and Engineering Chemistry. vol. 48, pp. 1342-1346 (1956) using polypropylene resin, such as "Dypro™ 50 MFR", commercially available from Fina Oil & Chemical Co., Cosden Div. The microfibers are 2 to 13 microns in size. The web had a basis weight of 185 $g/m^2$, a density of 0.0451 $g/cm^3$, a solidity of 5.0%, a void volume of 21.1 $cm^3/g$, and a void fraction of 95.1%.

The microfiber webs are then formed into particles having a size of less than 2 cm average diameter by milling on a hammer mill, a cryogenic mill, or a shredder, or by divellicating. The microfiber webs are divellicated as described in U.S. Patent No. 4,813,948, using a lickerin having a tooth density of 6.2 teeth/$cm^2$ and a speed of 900 rpm to produce microfiber microwebs having an average nuclei diameter of 0.5 mm and an average microweb diameter of 1.5 mm. Such divellicating produces microwebs having a relatively dense nucleus with fibers and fiber bundles extending therefrom. The nucleus of the microfiber microwebs is preferably in the range of 0.07 to 10.0 mm., more preferably 0.1 to 5 mm.

In a particularly preferred embodiment, about 35 grams of the divellicated microfiber microweb is placed into the column of a compression cylinder having a diameter of 6.07 cm (2.35 inches) whereupon it is molded into a plug of cylindrical shape 6.07 cm (2.35 inches) in diameter and 1.91 cm (0.75 inches) in height. The compression cylinder molds the microfiber microweb under a pressure of 4.2 MPa for a duration of 30 seconds. The plug is then dropped into the 208 x 108 size container prior to the removable lid being sealed to the top. Once inside the container, the plug of divellicated microfiber microweb recovers to fill the dimensions of the container in the diameter direction, whereupon the plug or packing material is in direct contact with the walls of the container.

Applicants have discovered that other suitable packing materials are useful in the practice of the present invention, provided they have a "packing factor" within a particular range. The packing factor is largely a function of the shape factor, particle size and void volume fraction of the packing material. The shape factor is defined as the sphericity squared times the particle diameter squared. To determine the packing factor first calculate the void volume fraction correction based on the absorbency of the packing material then multiply the void volume fraction correction by the shape factor. The void volume fraction correction is $(\varepsilon_w^3/(1-\varepsilon_w)^2)$, where $\varepsilon_w$ is the average porosity or void volume fraction between the unexposed and the steam exposed packing material. Further details regarding these values appear in McCabe and Smith, "Unit Operations of Chemical Engineering", 3rd Edition, McGraw Hill (1976). Packing materials useful in the practice of the present invention have packing factors generally in the range of 1.0 x $10^{-8}$ $cm^2$ to 1.3 x $10^{-5}$ $cm^2$, preferably in the range of 2.5 x $10^{-8}$ $cm^2$ to 3.0 x $10^{-6}$ $cm^2$.

Between the top end of the packing material and the lid 22 is a plug 28, having a recess 29 cut through it. The plug 28 can be made by injection molding or extruding suitable materials, including polycarbonate, polyamides, polymethylpentenes and various polyesters. Preferably plug 28 is made of polypropylene and plug 28 provides protection for the biological indicator 23 from breakage during shipping and sterilization, and also displaces the air, which would otherwise be present, above the packing material 26. Plug 28 preferably occupies that portion of the container not occupied by the packing material or biological indicator. In a preferred embodiment where the container is a 208 x 108 aluminum can, plug 28 is 1.27 cm high and 6.03 cm in diameter. The recess is adapted to receive a biological indicator device generally designated as 23. Plug 28 additionally has an indentation 11. This indentation is dimensioned to accommodate the inward movement of tab ring 24 when it is pulled to open lid 22.

The biological indicator 23 is preferably a unitary biological indicator, i.e., an indicator containing both the test microorganism and the nutrient growth medium. Exemplary biological indicators are described in U.S. Patent Nos. 3,239,429; 3,440,144; 3,661,717; 4,291,122; 4,304,869; 4,416,984; 4,461,837; 4,528,268; 4,579,823; 4,580,682; 4,596,773; and 4,717,661. A particularly preferred biological indicator is described and claimed in U.S. Patent No. 3,661,717, and is commercially available as "Attest[R] Biological Indicator" from 3M.

In the preferred embodiment illustrated in Fig. 1, a chemical indicator sheet 31, for detecting the penetration of the sterilant, is disposed within the container 12 between the plug 28 and the removeable lid 22. The chemical indicator will provide an immediate reading before the operator has an opportunity to incubate the biological indicator. The chemical indicator sheet of the present invention is comprised of an outer sheet 33 in the form of a disk co-extensive with and adhesively attached to the inside of the lid 22 such that the chemical indicator sheet can be removed for examination simultaneously with the removal of the lid. The chemical indicator sheet also has an inner sheet 35 in overlying relation to the outer sheet 33. The inner sheet is hinged to and detachable from the outer sheet such that when the test is completed, the inner sheet can be detached from the outer sheet and saved for record-keeping purposes. A chemical indicator ink 37 is present on the side of the inner sheet 35 in contact with the packing material. There is no need to separate the chemical indicator from the packing material by means of a separate container or condensate impermeable membrane.

Chemical indicator sheet 31 is of a type known in the art. Inner sheet 35 is made from a paper which has printed, on the side in contact with the packing material 26, a steam sensitive ink (not shown) in a test pattern designed to cover a substantial portion of the recess 29. The ink areas of the sheet are adapted to change color upon exposure to steam at a desired temperature for a desired period. The color change from white to black occurs over a period of time so that insufficiency of steam exposure may result in only partial development of the ink. This partial change may result in white or lightened areas, visible on the test sheet. Steam sensitive inks of this type are generally known in the art. Preferably the steam sensitive ink is an inorganic lead salt. A particularly preferred chemical indicator ink and paper substrate is commercially available as "Incheque ™ Type 1229S Indicator Sheet" from 3M Health Care, Loughborough, U.K. Other preferred chemical indicators are described in U.S. Patent No. 3,862,824, U.S. Patent No. 3,386,807, U.S. Patent No. 3,523,011, U.S. Patent No. 4,382,063 and U.K. Patent No. 1,458,553.

If ethylene oxide is used as the sterilant, the test pack 10 is equipped with an ethylene oxide chemical indicator sheet in place of the steam chemical indicator sheet. Useful ethylene oxide chemical indicator sheets are described in U.S. Patent Nos. 3,098,754; 3,258,312; 3,627,469; 3,852,034; 4,015,937; 4,094,642; 4,168,779; and U.K. Patent No. 1,370,470. Particularly preferred ethylene oxide chemical indicator sheets are commercially available as "Incheque™ Type 1202 Internal Chemical Indicator" and "Comply ™ 1251 Chemical Indicator Strip", from 3M. The color change in the ethylene oxide chemical indicator sheets can be evaluated after the sterilization process by using a color specific system such as "Pantone[R] Color Specifier" commercially available from Pantone, Inc., Moonachie, New Jersey.

Referring now to Fig. 1, the biological indicator 23 is shown having an outer container in the shape of cylindrical tube 30, having substantially gas non-absorptive and liquid impermeable walls 32 and an open end 34. Tube 30 contains a carrier 36, such as a strip of filter paper, bearing a predetermined amount of viable microorganisms. Tube 30 also includes a normally sealed, pressure-openable inner container 38, such as a frangible glass ampule, containing an aqueous nutrient growth medium 40. The aqueous nutrient medium is capable, with incubation, of promoting growth of any viable test microorganisms not killed during the sterilization cycle when contacted therewith, and preferably contains a microbial growth indicator which provides a change in color of the solution if viable microorganisms are present, indicating an inadequate cycle. The inner container 38 is preferably snugly retained within the outer container 30 so that very little of the volume of the outer container remains unoccupied. The glass ampoule 38 is separated from the wall 32 of the tube 30 by the filter paper carrier 36. The open end 34 of the tube 30 is provided with a gas-transmissive bacteria-impermeable closure member 42, such as a sheet. The sheet 42 may be sealed to the open end 34 of the tube 30 by, e.g., heat or adhesive sealing, or by means of a closure device 46, such as a cap, which has an aperture 48 therethrough. During sterilization, sterilant which penetrates the packing material along with condensate or noncondensable gases permeates the sheet 42 and passes through the interior of the outer container to contact the carrier 36.

The biological indicator device 23 may be easily assembled by sequentially inserting into the open end 34 of the tube 30 the carrier 36 and the frangible glass ampule 38, and sealing the open end 34 of the tube with the sheet 42 by placing sheet 42 over open end 34 and then placing cap 46 over sheet 42, in closing engagement with tube 30.

Outer container 30 is made from material which will withstand the high temperatures encountered in steam sterilizers. Conventional steam sterilizers generally reach temperatures on the order of 121°C-135°C. Additionally, the walls of the container 30 must be substantially impermeable to gases and liquids. Outer container 30 which contains carrier 36 which is coated with viable microorganisms, is preferably translucent (including "transparent") so that a change in fluorescence or color may be visually observed without disassembling the indicator device. Preferably, also, the outer container 30 is sufficiently deformable so that the pressure-openable inner compartment 38 is ruptured when the outer compartment 30 is deformed, by using external pressure. Container 30 can be made by injection molding or extruding suitable materials, including polycarbonate, polypropylene, polyamides, polymethylpentenes and various polyesters. Polypropylene is the preferred material. These materials are sufficiently temperature resistant to withstand steam or dry heat sterilization cycles, non-absorbent of gaseous sterilizing media, liquid-impermeable, translucent or transparent and deformable.

The closure device 46 can be made from any material that will withstand the sterilization temperatures. As in the case of the container 30, suitable materials include polycarbonate, polypropylene, polyamides, polymethylpentenes and various polyesters, with polypropylene being preferred.

The microorganisms which are employed in the present invention normally are carried on a suitable carrier 36. It is contemplated, however, that the microorganism may be carried by the inner walls of the outer container 30, or the outer walls of the inner container 38. The carrier 36 preferably is water-absorbent, such as filter paper, and should not inhibit microorganism growth. Sheet-like materials such as cloth, nonwoven polypropylene, rayon or nylon, and microporous polymeric materials are especially preferred. However, metal foil substrates, for example, aluminum or stainless steel may be used, as well as substrates of glass (e.g., glass beads or glass fibers), porcelain, or plastic. Additionally, the carrier 36 can be constructed of a combination of materials such as paper secured to a plastic or glass backing strip.

Microorganisms which may be employed in the present invention are those conventionally used microorganisms which are generally many times more resistant to the sterilization process being employed than most organisms encountered in natural contamination. Favorable results have been obtained with bacteria and fungi which exist in both "spore" and "vegetative" states. The bacterial spore is recognized as the most resistant form of microbial life. It is the life form of choice in all tests for determining the sterilizing efficacy of devices, chemicals and processes. Spores from Bacillus and Clostridia species are the most commonly used to monitor sterilization processes utilizing saturated steam, dry heat, and ethylene oxide.

Particularly preferred microorganisms commonly used to monitor sterilization conditions include Bacillus stearothermophilus and Bacillus subtilis. Bacillus stearothermophilus is particularly useful to monitor sterilization under steam sterilization conditions. Bacillus subtilis is particularly useful to monitor conditions of gas and dry heat sterilization.

Inner container 38 contains an aqueous solution of nutrient growth media. The types of nutrient media usefully employed in the present invention are widely known to the art. Examples of preferred nutrient media are aqueous solutions of soybean-casein digest broth, fluid thioglycollate and Dextrose Tryptone (Difco Laboratories, Inc.). A modified tryptic soy broth base, without glucose, is especially preferred. To avoid contamination, such aqueous nutrient media normally is sterilized after having been placed in the inner compartment. Commonly known microbial growth indicators, which change color in the presence of viable microorganisms, are preferably present in at least one of the containers. The growth indicator material preferably is soluble in, and imparts color (upon microorganism growth) to, the aqueous nutrient medium so that a change in color may be easily observed through the translucent walls of the outer container. Growth indicator materials which may be employed in the present invention are well known to the art and include pH-sensitive dye indicators (such as bromthymol blue, bromcresol purple, phenol red, etc.), oxidation-reduction dye indicators (such as methylene blue, etc.). Such materials commonly undergo changes in color in response to a phenomenon of microorganism growth, such as changes in pH, oxidation-reduction potentials, etc.

The inner container 38 which contains the aqueous nutrient medium, is of material which is impermeable to gases and liquids and is capable of being opened upon the application of pressure thereto (i.e., "pressure openable") to permit the nutrient medium 40 to enter the outer container. The inner container is preferably of frangible material, such as glass, and, as mentioned above, is preferably snugly carried within the outer container in coacting relationship therewith to permit breakage or crushing of the inner container when the outer container is deformed. In another embodiment, the inner container may be sealed with a plug such that the plug is expelled to release the contents of the inner container upon application of pressure. In still another embodiment, the closure 46 may include an ampoule crushing device, as shown in U.S. Pat. 4,304,869, wherein the closure has tabs extending from the bottom of the closure device which upon depression of the closure device serve to crush the ampoule. Similarly, the device of the present invention may be used in a system having an ampoule crushing pin disposed in the bottom of the outer container 30.

The outer container 30 has at least one opening therein to permit the sterilant (e.g., steam, ethylene oxide) which has penetrated the packing material to contact the source of active enzyme during sterilization. This opening is normally closed or plugged with a gas-transmissive bacteria-impermeable means. Suitable means include closure member 42, made of fibrous materials such as cotton, glass wool, cloth, nonwoven webs made from polypropylene, rayon, polypropylene/rayon, nylon, glass or other fibers, filter papers, and microporous hydrophobic or hydrophilic films, open celled polymeric foams, and semi-permeable plastic films such as those described in U.S. Pat No. 3,346,464. Fibrous or cellular materials are preferred because of the ease with which such materials transmit sterilizing gases. In effect, the fibrous or cellular closure members serve as filters for bacteria and fungi and hence should have pore sizes no larger than 0.5 microns (e.g. be capable of preventing passage therethrough of particles having dimensions larger than 5 microns). Alternatively, the closure means may be a tortuous pathway that is bacteria-impermeable, such as that described in U.S. Patent No. 4,461,837, and in commonly assigned United States Patent No. 4,883,641, issued November 28, 1989.

In a particularly preferred embodiment of the present invention, the biological indicator 23 employs an enzyme whose activity can be correlated with the viability of at least one microorganism commonly used to monitor sterilization efficacy, hereinafter referred to as a "test microorganism". The enzyme, following a sterilization cycle which is sublethal to the test microorganism, remains sufficiently active to react with an enzyme substrate in a relatively short period of time, e.g., normally eight hours or less. However, the enzyme is inactivated or appreciably reduced in activity following a sterilization cycle which is lethal to the test microorganism. In this particularly preferred embodiment, the unitary sterility indicator 23 includes a source of active enzyme contained in the outer container 30, and a substrate system for that enzyme in the inner or outer container. The enzyme substrate system is capable of reacting in the presence of an aqueous reaction medium with active enzyme to produce a detectable enzyme-modified product. The use of such enzymes to monitor sterilization efficacy is described in commonly assigned U.S. Patent application entitled "Rapid Method for Determining Efficacy of a Sterilization Cycle and Rapid Read-Out Biological Indicator", U.S. Serial No. 277,305, filed November 29, 1988, and European Publication No. 0 371 682, filed November 22, 1989.

The test pack 10 as described, is loaded in a steam or gas sterilization chamber. In prevacuum steam sterilizers, a vacuum is drawn to evacuate the air in the sterilizer to a level low enough to be acceptable for a proper sterilization cycle. Steam is then introduced into the sterilizer. The steam will enter the holes 18 on the bottom wall 16 of the test pack and pass upwardly through the packing material 26. The steam is condensed and then adsorbed on the fibrous mass of the packing material. Any noncondensable gas which was not drawn out during the vacuum, or which was mixed with the steam, will be released and trapped as air pockets in the packing material. Any noncondensed gas present in the packing material will act as a barrier preventing the steam from affecting the biological indicator. Also, the failure of the steam to penetrate the packing material due to other reasons, such as improperly operating the sterilization cycle for an insufficient length of time at a particular temperature, will prevent the steam from affecting the biological indicator.

Similarly, in gravity steam sterilizers, the incoming steam displaces the air disposed in the packing material. If an inadequate cycle has taken place, the steam does not sufficiently displace the air thereby causing the air to insulate the indicator and preventing at least portions of the indicator from being affected by the steam.

In ethylene oxide sterilizers, the ethylene oxide gas (optionally, in combination with other inert, diluent gases such as carbon dioxide and halocarbon gases) is introduced into the chamber and allowed to mix with water vapor. If the proper conditions of ethylene oxide concentration, relative humidity, time and temperature are not met, or if the ethylene oxide does not penetrate the packing material, all of the test microorganisms present in the biological indicator will not be killed.

Once the sterilization cycle is completed, the disposable test pack 10 is removed from the sterilizer and the lid 22 is removed to provide quick access to the biological indicator device 23. The biological indicator is then treated with the nutrient growth solution and/or enzyme substrate to reveal either no color change or no fluorescence, which would indicate that the enzymes or living microorganisms of the biological indicator were inactivated as a result of a successful cycle, or a complete color change or fluorescence, which would indicate an unacceptable sterilization cycle due to the failure of the sterilant to properly penetrate the packing material.

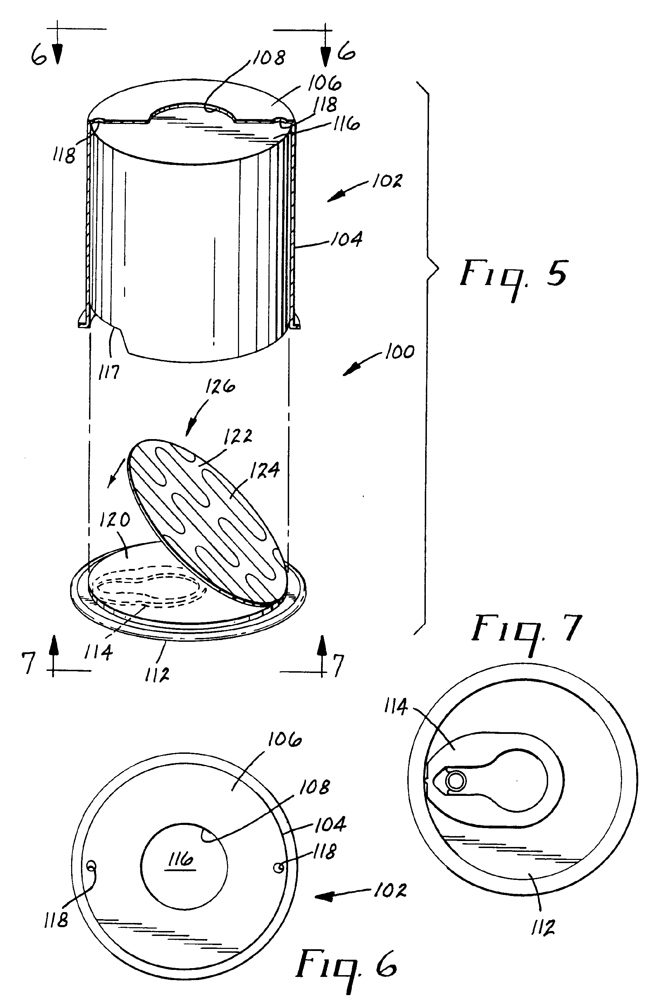

An alternative embodiment of the present invention is generally shown as 100 in Figs. 5-7. Referring to Fig. 5 in particular, the disposable test pack includes a vertically extending container 102 having a tubular side wall 104 and a top wall 106. The container is preferably 5.56 cm (2 3/16 inches) in height and 6.35 cm (2 1/2 inches) in diameter, designated by Central States Cans Co. as size 208 x 203. However, the container of the present invention can be varied in size as disclosed in the embodiment illustrated by Figs. 1-4. The container is preferably made from aluminum but the invention does allow for the use of other suitable impermeable materials. The top wall of the container has a hole 108 which allows the ingress of the sterilant during the sterilization cycle. The hole of the test pack of this embodiment preferably has a diameter of 2.22 cm (7/8 in). However, it should be appreciated that the hole having a diameter in the range of 0.32 cm to 2.54 cm can be provided for the given suggested container sizes. The bottom wall of the test pack container comprises a lid 112 which is provided with a pull tab ring 114. The lid, which seals the lower portion of the container, can be easily removed to permit easy access to the inside of the disposable test pack after sterilization by simply pulling on the tab ring and lifting the lid away from the container. Thus, the container, which is similar to the one used in the first embodiment of the invention, provides an economical, small, and easy to use disposable test pack.

The container is filled with a porous, fibrous mass packing material 116, such as the materials disclosed for use in the first embodiment of the present invention. The packing material is prepared and placed in the container as described in the first embodiment, with the exception that the volume of the entire can is filled with the packing material. In a particularly preferred embodiment, 60 grams of the divellicated polypropylene microfiber microweb described hereinabove is used to fill container 102. The packing material consists of two sections with different densities. The section closest to hole 108 weighs 30 grams and is compressed to initially 5.97 cm in diameter and 3.97 cm in height. The section closest to the lid 112 weighs 30 grams and is compressed to initially 5.97 cm in diameter and 1.59 cm in height. The packing material once placed in the container 102 (size 208 x 203) expands to fill the container. The compressed polypropylene blown microfiber packing material when used in the disposable test pack of the present invention provides an excellent challenge to the penetration of the sterilant for determining the efficacy of the sterilization cycle. However, other suitable packing materials which provide the necessary challenge such as the ones described for use in the first embodiment of the invention, can be used. Cut within the packing material is indentation 117, which is dimensioned and shaped to accommodate the inward movement of tab ring 114 when it is pulled to open lid 112.

A chemical indicator sheet 126, for detecting the penetration of the sterilant, is disposed within the container 102 between the lower end of the packing material 116 and the lid 112 or bottom end of the container. The chemical indicator sheet of the present invention is comprised of an outer sheet 120 in the form of a disk co-extensive with and attached to the inside of the lid such that the chemical indicator sheet can be removed for examination simultaneously with the removal of the lid. The chemical indicator sheet also has an inner sheet 122 in overlying relation to the outer sheet. The inner sheet is hinged to and detachable from the outer sheet such that when the test is completed, the inner sheet can be detached from the outer sheet and saved for record-keeping purposes. A chemical indicator material 124 of the type described hereinabove is placed on the side of the inner sheet in contact with the packing material. There is no need to separate the chemical indicator from the packing material by means of a separate container or condensate impermeable membrane.

In another embodiment of the device depicted in Figs. 5-7, if ethylene oxide is used as the sterilizing medium in a sterilization chamber, the disposable test pack 100 is equipped with ethylene oxide indicator sheets in place of the steam chemical indicator sheets.

In the embodiment depicted in Figs. 5-7, two small openings 118 are provided on opposite edges of the top wall 106 of the test pack container 102. The openings vent steam or gas to relieve the temperature and pressure of the test pack, thereby preventing the sterilant from rapidly escaping or bursting out of the test pack if the lid 112 is removed soon after the sterilization process. The vents are preferably no larger than 0.16cm (1/16 in) in diameter, and are preferably 0.08cm (1/32 in) in diameter.

During a prevacuum steam sterilization cycle, steam enters the disposable test pack 100 through the hole 108 in the top wall 106 of the container 102 and comes in contact with the packing material 116. The packing material then extracts the latent heat from the steam causing it to collapse or condense where it is adsorbed on the packing material. The temperature of the packing material increases as the latent heat of the steam is extracted by the packing material. As steam continues to enter the test pack, a penetration gradient of steam occurs along the packing material towards the chemical indicator. Any noncondensable gas or air which is present or which is mixed with the steam will be trapped as air pockets in the packing material. The air pockets, if any, will prevent the temperature sensitive chemical indicator 126 from contacting the heat and steam thereby preventing the chemical indicator from exhibiting a completely uniform color change. If no air pockets are present to prevent the penetration of the steam through the packing material, the heat and steam will affect the chemical indicator sheet 126. Also, another fault condition which will result in the chemical indicator from being unaffected is the failure to run the sterilization cycle for a proper length of time at a necessary temperature in order to allow the steam to completely penetrate the packing material.

Once the sterilization process is complete, the lid 112 is easily removed providing quick access to the chemical indicator sheet 126. The chemical indicator sheet is then examined to determine whether the sterilization was satisfactory or whether unacceptable levels of noncondensable gas were present. A uniform color change in the chemical indicator sheet is indicative of an adequate sterilization cycle. A chemical indicator sheet which reveals no or a non-uniform color change is an indication that noncondensable gas was present in the sterilizer or that the sterilant did not effectively penetrate the packing material.

Another feature of the embodiment depicted in Figs. 5-7 of the invention allows for the disposable test pack to be inverted or turned on its side for prevacuum and gravity steam sterilizers, wherein the hole for the ingress of the sterilant is at the bottom end or on the side of the disposable test pack. The chemical indicator will indicate whether the sterilant used in the sterilizer has sufficiently displaced the air in the test pack or whether a sufficient amount of air was not displaced by the sterilant thereby resulting in an inefficacious sterilization cycle.

The test pack containers 10 and 100 can also be provided with a chemical indicator on the outside of their sidewalls to readily identify whether the test packs have already been subjected to a sterilization cycle. This eliminates the need to remove the container lids to examine the indicators inside the test packs to determine if they have been used. This also avoids the possibility of wasting a test pack which has not been used, or using a test pack which has already been through a cycle in a sterilization chamber.

It should be appreciated from the foregoing description that the present invention provides improved disposable test packs using chemical and biological indicators for determining the efficacy of a sterilization cycle in prevacuum, gravity of ethylene oxide sterilizers. the disposable test packs provide either chemical or biological indicators in containers which are relatively inexpensive and which are easy to use. The containers have lids which are easily removed to allow access to the indicators within the test packs. The disposable test packs do not require any means to separate the indicators from any condensed steam that forms in the packing material, and the indicators may be provided in the same container holding the packing material.

The test packs of the present invention have been described primarily with reference to sterilizing media such as ethylene oxide, steam and the like. The test packs are not, however, limited to these uses, and may as well be used to indicate the efficacy of other sterilizing media, such as dry heat, radiation, propylene oxide, methyl bromide, ozone, chlorine dioxide, formaldehyde, and other gaseous and liquid agents.

## Claims

1. A disposable test pack (10) for determining the efficacy of a sterilization cycle in a sterilization chamber characterized in that said test pack (10) comprises:

   I) a vertically extending container (12) made of gas and liquid-impermeable material and having
      a) a top wall (20),
      b) sidewalls (14), and
      c) a bottom wall (16), wherein said bottom wall (16) has at least one hole (18) therethrough for the ingress of sterilant;

   II) porous fibrous packing material (26) at least partially filling said container (12) for challenging the penetration of sterilant by defining a restricted pathway which impedes the flow of the sterilant through said container (12) during the sterilization cycle;

   III) an indicator (23 or 31) located on the top of the packing material (26) for indicating whether the sterilant has successfully penetrated through said packing material (26) and to what extent, thereby determining the efficacy of the sterilization cycle; and

   IV) a lid (22) sealing the top wall (20) of said container (12), overlying said indicator (23 or 31), said lid (22) being removable to enable exposure of said indicator (23 or 31) after the sterilization cycle.

2. The disposable test pack (10) of claim 1, further characterized in that said indicator includes at least one biological (23) or chemical (31) indicator.

3. A disposable test pack (100) for determining the efficacy of a sterilization cycle in a sterilization chamber characterized in that said test pack (100) comprises:

   I) a vertically extending container (102) made of gas and liquid-impermeable material and having a first end wall (106) and a second end wall (112) opposite said first end wall (106), and sidewalls (104) separating said first (106) and second walls (112), wherein said first end wall (106) has at least one hole (108) therethrough for the ingress of sterilant

   II) porous fibrous packing material (116) at least partially filling said container (102) for challenging the penetration of the sterilant by defining a restricted pathway which impedes the flow of the sterilant through said container (102) during the sterilization cycle;

   III) a chemical indicator (126) located between said second wall (112) and said packing material (116), said chemical indicator (126) having areas adapted to change color in response to the presence of a sterilant under preselected sterilization conditions and;

   IV) and a lid (112) sealing said second wall of said container (102) and overlying said chemical indicator sheet (126), said lid (112) being removable to enable exposure of said chemical indicator (126) after the sterilization cycle.

4. The disposable test pack of claims 1 or 3 further characterized in that said packing material (26, 116) is compressed divellicated polypropylene blown microfiber microweb.

5. The disposable test pack of claim 3, further characterized in that said chemical indicator (126) is comprised of:

an outer sheet (120) generally co-extensive with said lid (112) and adhesively secured thereto;

an inner sheet (122) integrally hinged to, and detachable from said outer sheet (120) in overlying relation thereto, said inner sheet (122) in direct contact with the packing material (116);

chemical indicator material (124) on said inner sheet (122) for indicating the penetration of the sterilant used during the sterilization cycle; and

whereby detachment of said lid (112) after sterilization simultaneously removes said chemical indicator sheet (126) from said container with said inner sheet (122) readily accessible for removal by tearing it from said outer sheet (120).

6. A disposable test pack (10) for determining the efficacy of a sterilization cycle in a sterilization chamber characterized in that said test pack (10) comprises:

I) a container (12) made of a gas and liquid-impermeable material and having a volumetric capacity in the range of 2.54 cm³ to 508 cm³, said container (12) having a first end wall (16) and a second end wall (20) opposite said first end wall (16), and sidewalls (14) separating said first (16) and second walls (20), wherein said first end wall (16) has at least one hole (18) therethrough for the ingress of sterilant, said holes (18) have a cumulative area of between 0.0254 cm² and 7.62 cm²;

II) porous, fibrous packing material (26) placed inside said container (12) for challenging the penetration of the sterilant by defining a restricted pathway which impedes the flow of the sterilant through said container (12) during the sterilization cycle, said packing material (26) having a packing factor characteristic in the range of $1.0 \times 10^{-8}$ cm² to $1.3 \times 10^{-5}$ cm²;

III) an indicator (23, 31) located within said container (12) on the top of said packing material (26) for indicating whether the sterilant has successfully penetrated through said packing material (26) and to what extent, thereby determining the efficacy of the sterilization cycle; and

IV) means (22) for opening said container (12) to expose said indicator (23, 31) after sterilization.

**Patentansprüche**

1. Einweg-Testpackung (10) zum Bestimmen des Erfolgs eines Sterilisationszyklus in einer Sterilisierkammer, dadurch gekennzeichnet, daß die Testpackung (10) aufweist:

I) einen sich vertikal erstreckenden Behälter (12), der aus gas- und flüssigkeitsundurchlässigem Werkstoff besteht und

a) eine Decke (20),

b) Seitenwände (14) und

c) einen Boden (16) aufweist, der von mindestens einem Loch (18) für den Eintritt von Sterilisationsmittel durchsetzt ist;

II) einen porösen faserigen Füllstoff (26), der den Behälter (12) wenigstens teilweise ausfüllt und das Eindringen des Sterilisationsmittels dadurch behindert, daß er einen verengten Kanal bildet, der während des Sterilisationszyklus das Strömen des Sterilisationsmittels durch den Behälter (12) hemmt;

III) einen oben auf dem Füllstoff (26) vorgesehenen Indikator (23 oder 31) zum Anzeigen, ob und in welchem Ausmaß des Sterilisationsmittel durch den Dichtstoff (26) hindurchgetreten ist, so daß der Erfolg des Sterilisiervorganges bestimmbar ist; und

IV) einen über dem Indikator (23 oder 31) liegenden Deckel (22), der die Decke (20) des Behälters (12) dicht abschließt und der nach dem Sterilisiervorgang zum Freiligen des Indikators (23 oder 31) abnehmbar ist.

2. Einweg-Testpackung (10) nach Anspruch 1, dadurch gekennzeichnet, daß der Indikator mindestens einen biologischen (23) oder chemischen (31) Indikator aufweist.

3. Einweg-Testpackung (100) zum Bestimmten des Erfolges eines Sterilisationszyklus in einer Sterilisationskammer, dadurch gekennzeichnet, daß die Testpackung (100) aufweist:

I) einen sich vertikal erstreckenden Behälter (102), der aus einem gas- und flüssigkeitsundurchlässigen Werkstoff besteht und eine erste Endwand (106), eine der ersten Endwand (106) und die zweite (112) Endwand voneinander trennende Seitenwände (104) besitzt, wobei die erste Endwand (106) von mindestens einem Loch (108) für den Eintritt von Sterilisationsmittel durchsetzt ist;

II) einen porösen faserigen Füllstoff (116), der den Behälter (102) wenigstens teilsweise ausfüllt und das Eindrigen des Sterilisationsmittels dadurch behindert, daß er einen verengten Kanal bildet, der während des Sterilisationszyklus das Strömen des Sterilisationsmittels durch den Behälter (102) hemmt;

III) einen zwischen der zweiten Wand (112) und dem Füllstoff (116) angeordneten chemischen Indikator (126), der Flächen hat, die geeignet sind, sich bei Vorhandensein eines Sterilisationsmittels unter vorgewählten Sterilisationsbedingungen zu verfärben; und

IV) einen über dem chemischen Indikatorblatt (126) angeordneten Deckel (112), der die zweite Wand des Behälters (102) dicht abschließt und zum Freilegen des chemischen Indikators (126) nach dem Sterilisationszyklus abnehmbar ist.

4. Einweg-Testpackung nach Anspruch 1 bis 3, dadurch gekennzeichnet, daß der Füllstoff (26, 116) ein gepreßter zerkleinerter (divellicated) Mikrovliesstoff aus geblasenen Polypropylen-Mikrofasern ist.

5. Einweg-Testpackung nach Anspruch 3, dadurch gekennzeichnet, daß der chemische Indikator (126) aufweist:

ein Außenblatt (120), das sich allgemein über dieselbe Fläche erstreckt wie der Deckel (112) und mit ihm verklebt ist;

ein Innenblatt (122), das über dem Außenblatt (120) liegt und einstückig an diesen angelenkt und von ihm ablösbar ist und das mit dem Füllstoff (116) direkt in Berührung steht;

eine auf dem Innenblatt (122) vorgesehene chemische Indikatorsubstanz (124) zum Anzeigen des Hindurchtretens des während des Sterilisationszyklus verwendeten Sterilisationsmittels;

so daß durch Abnehmen des Deckels (112) nach dem Sterilisieren gleichzeitig such das chemische Indikatorblatt (126) aus dem Behälter entfernt wird und das Innenblatt (122) durch Abreißen von dem Außenblatt (120) leicht für das Herausnehmen zugänglich ist.

6. Einweg-Testpackung (10) zum Bestimmen des Erfolgseines Sterilisationszyklus in einer Sterilisierkamme, dadurch gekennzeichnet, daß die Testpackung (10) aufweist:

I) einen aus gas- und flüssigkeitsundurchlässigem Werkstoff bestehenden Behälter (12) der eine Volumenkapazität von 2,54 cm$^3$ bis 508 cm$^3$ hat und eine erste Endwand (16), eine der ersten Endwand (16) gegenüberliegende zweite Endwand (20) und die erste (16) und die zweite (20) Endwand voneinander trennende Seitenwände (14) besitzt, wobei die erste Endwand (16) von mindestens einem Loch (18) für den Eintritt von Sterilisationsmittel durchsetzt ist und die genannten Löcher (18) eine Gesamtfläche von 0,0254 cm$^2$ bis 7,62 cm$^2$ haben;

II) einen in dem Behälter (12) angeordneten, porösen faserigen Füllstoff (26) der das Eindrigen des Sterilisationsmittels dadurch behindert, daß er einen verengten Kanal bildet, der während des Sterilisationszyklus das Strömen des Sterilisationsmittels durch den Behälter (12) hemmt; wobei der Füllstoff einen Füllfaktor im Bereich von $1,0 . 10^{-8}$ bis $1,3 . 10^{-5}$ cm$^2$ hat.

III) einen in dem Behälter (12) oben auf dem Füllstoff (26) angeordneten Indikator (23, 31) zum Anzeigen ob und in welchem Ausmaß das Sterilisationsmittel während des Sterilisationszyklus durch den Füllstoff (26) hindurchgetreten ist, so daß der Erfolg des Sterilisationszyklus bestimmbar ist; und

IV) Mittel (22) zum Öffnen des Behälters (12) zum Freilegen des Indikators (23, 31) nach der Sterilisation.

## Revendications

1. Dispositif de test jetable (10) pour déterminer l'efficacité d'un cycle de stérilisation dans une chambre de stérilisation, caractérisé en ce que ledit dispositif de test (10) comprend :

I) un récipient vertical (12) fabriqué en une matière imperméable aux gaz et aux liquides et comportant :

(a) une paroi supérieure (20),

(b) des parois latérales (14), et

(c) une paroi inférieure (16), ladite paroi inférieure (16) étant traversée par au moins un trou (18) pour l'introduction d'un agent stérilisant ;

II) une matière poreuse et fibreuse de garnissage (26) remplissant au moins partiellement ledit récipient (12) de manière à résister à la pénétration de l'agent stérilisant , par définition d'un passage restreint qui freine l'écoulement du stérilisant à travers ledit récipient (12) pendant le cycle de stérilisation ;

III) un indicateur (23 ou 31) placé sur le dessus de la matière de garnissage (26),pour indiquer si l'agent stérilisant a pénétré avec succès dans la dite matière de garnissage (26) et dans quelle mesure, ce qui détermine l'efficacité du cycle de stérilisation ; et

IV) un couvercle (22) fermant la paroi supérieure (20) dudit récipient (12) et placé au-dessus dudit indicateur (23 ou 31), ledit couvercle (22) pouvant être enlevé pour permettre de découvrir ledit indicateur (23 ou 31) après le cycle de stérilisation.

2. Dispositif de test jetable (10) suivant la revendication 1, caractérisé en outre en ce que ledit indicateur comprend au moins un indicateur biologique (23) ou chimique (31).

3. Dispositif de test jetable (100) pour déterminer l'efficacité d'un cycle de stérilisation dans une chambre de stérilisation, caractérisé en ce que ledit dispositif de test (100) comprend :

I) un récipient vertical (102) fabriqué en une matière imperméable aux gaz et aux liquides et comportant une première paroi d'extrémité (106) et une deuxième paroi d'extrémité (112) opposée à ladite première paroi d'extrémité (106), et des parois latérales (104) séparant lesdites première (106) et deuxième (112) parois, ladite première paroi d'extrémité (106) étant traversée par au moins un trou (108) pour l'entrée d'agent stérilisant ;

II) une matière poreuse et fibreuse de garnissage (116) qui remplit au moins partiellement le dit récipient (102) pour s'opposer à la pénétration de l'agent stérilisant, par définition d'un passage restreint qui freine l'écoulement de l'agent stérilisant à travers ledit récipient (102) pendant le cycle de stérilisation ;

III) un indicateur chimique (126) placé entre ladite deuxième paroi (112) et ladite matière de garnissage (116), ledit indicateur chimique (126) ayant des régions qui changent de couleur en réponse à la présence d'un agent stérilisant dans des conditions de stérilisation prédéterminées ; et

IV) un couvercle (112) qui ferme la dite deuxième paroi du dit récipient (102) et est placé au-dessus de ladite feuille d'indicateur chimique (126), ledit couvercle (112) pouvant être enlevé pour permettre l'exposition du dit indicateur chimique (126) après le cycle de stérilisation.

4. Dispositif de test jetable suivant les revendications 1 ou 3, caractérisé en outre en ce que la dite matière de garnissage (26,116) est une microfeuille de microfibres soufflées de polypropylène, déchiquetée et comprimée.

5. Dispositif de test jetable suivant la revendication 3, caractérisé en outre en ce que ledit indicateur chimique (126) est composé de :

une feuille extérieure (120) sensiblement coextensive avec ledit couvercle (112) et collée à celui-ci ;

une feuille intérieure (122) intégralement articulée à ladite feuille extérieure (120) et détachable de celle-ci, en superposition mutuelle, ladite feuille intérieure (122) étant en contact direct avec la matière de garnissage (116) ;

un indicateur chimique (124) appliqué sur la dite feuille intérieure (122) pour indiquer la pénétration de l'agent stérilisant utilisé pendant le cycle de stérilisation ;

de sorte que le détachement dudit couvercle (112) après la stérilisation enlève simultanément ladite feuille d'indicateur chimique (126) dudit récipient, la dite feuille intérieure (122) étant facilement accessible pour enlèvement par déchirure de ladite feuille intérieure par rapport à ladite feuille extérieure (120).

6. Dispositif de test jetable (10) pour déterminer l'efficacité d'un cycle de stérilisation dans une chambre de stérilisation, caractérisé en ce que ledit dispositif de test (10) comprend :

I) un récipient (12) fabriqué en une matière imperméable aux gaz et aux liquides et ayant une capacité comprise entre 2,54 cm³ et 508 cm³, ledit récipient (12) comportant une première paroi d'extrémité (16) et une deuxième paroi d'extrémité (20) opposée à ladite première paroi d'extrémité (16), et des parois latérales (14) séparant lesdites première (16) et deuxième (20) parois, ladite première paroi d'extrémité (16) ayant au moins un trou traversant (18) pour l'entrée d'agent stérilisant, lesdits trous (18) ayant une section cumulée comprise entre 0,0254 cm² et 7,62 cm² ;

II) une matière poreuse et fibreuse de garnissage (26) placée à l'intérieur dudit récipient (12) pour s'opposer à la pénétration de l'agent stérilisant, par définition d'un passage restreint qui freine l'écoulement de l'agent stérilisant à travers ledit récipient (12) pendant le cycle de stérilisation, ladite matière de garnissage (26) ayant un facteur de compactage compris entre $1,0 \times 10^{-8}$ cm² et $1,3 \times 10^{-5}$ cm² ;

III) un indicateur (23,31) placé à l'intérieur dudit récipient (12), sur le dessus de ladite matière de garnissage (26), pour indiquer si l'agent stérilisant a correctement traversé ladite matière de garnissage (26) et dans quelle mesure, ce qui détermine l'efficacité du cycle de stérilisation ; et

IV) des moyens (22) d'ouverture dudit récipient (12) pour découvrir ledit indicateur (23,31) après la stérilisation.

Fiq 1

Fig. 2

Fig. 3

Fig. 4

Fig. 5

Fig. 7

Fig. 6